# EUROPEAN PATENT APPLICATION

(11) **EP 4 596 527 A1**
(43) Date of publication of application: **06.08.2025**
(21) Application number: 23869780.9
(22) Date of filing: 27.06.2023
(51) Int. Cl.: C07C 67/38, C07C 69/40, C07C 69/34, C07C 69/612, C07C 69/602, C07C 69/65, C07C 69/608, C07C 69/38, C07C 69/44, C07C 69/48, C07C 69/63

(54) **METHOD FOR PREPARING DIESTER BY OXIDATIVE CARBONYLATION OF OLEFIN AND CARBON MONOXIDE**

(30) Priority: 26.09.2022 CN 202211169758
(71) Applicant: Sun Yat-Sen University, Guangzhou, Guangdong 510275 (CN)
(72) Inventor: LI, Suhua, Guangzhou, Guangdong 510275 (CN); MENG, Bingyin, Guangzhou, Guangdong 510275 (CN)
(74) Representative: Kröncke, Rolf
(86) International application number: PCT/CN2023/102612
(87) International publication number: WO 2024/066532

(57) **Abstract**

A method for preparing a diester by oxidative carbonylation of an olefin and carbon monoxide, which comprises the following steps: carrying out an oxidative carbonylation reaction on olefin and carbon monoxide in an oxygen-containing atmosphere and in the presence of a catalyst to obtain a 1,4-diester-based compound; the catalyst comprises a procatalyst, a pro-oxidant, an acid, and a dehydrating agent; and the procatalyst is a substance comprising the element Pd; the pro-oxidant comprises one or more among copper salt, iron salt, and iron powder; the acid comprises Lewis acid and/or protonic acid; and the dehydrating agent comprises one or more among a ketal, an acetal, orthoformate, and silicate. The present method has high catalytic efficiency, high selectivity, and a high conversion rate; oxygen or air is used as an oxidizing agent; the catalyst is a green, low-carbon, environment-friendly catalytic system that can be reused, is low-cost, and produces essentially no waste in the production process; and the oxidative carbonylation reaction of the catalyzed olefin and CO prepares the 1,4-diester compound.

## Description

This application claims the priority to Chinese Patent Application No. 202211169758.9, filed on September 26, 2022 with the China National Intellectual Property Administration, titled "METHOD FOR PREPARING DIESTER BY OXIDATIVE CARBONYLATION OF OLEFIN AND CARBON MONOXIDE", which are incorporated herein by reference in entirety.

### FIELD

The present disclosure belongs to the technical field of organic synthesis and particularly relates to a method for preparing a diester by oxidative carbonylation of an olefin and carbon monoxide.

### BACKGROUND

Oxidative carbonylation refers to the reaction of carbon monoxide with one or more of a nucleophile, an unsaturated hydrocarbon and the like in the presence of an oxidant to produce a compound such as an ester, a carbonate, and a urea. In the oxidative carbonylation reaction of olefin and carbon monoxide for preparation of a succinate ester, Pd is commonly required as a catalyst (usually starting from the divalent state). During the process of obtaining a succinate ester in the reaction, one molecule of water is simultaneously generated, and Pd is reduced to the zero-valent state. At this time, an oxidant/co-oxidant is needed to oxidize Pd back to the divalent state to complete one catalytic cycle.

Patent US 3530168 discloses a phosphine ligand-promoted oxidative carbonylation reaction, wherein the reaction rate had a certain increase (the turnover frequency (TOF) was 145/h) but the total turnover number (TON) was only 72, the selectivity of the reaction was poor, the selectivity of dimethyl succinate was about 60%, and a large amount of acrylate by-product was generated in the reaction.

The existing oxidative carbonylation reaction of olefins with CO has many problems, such as low catalytic efficiency (requiring the use of a large amount of expensive metal catalyst Pd), poor selectivity, low conversion rate, or the need for equivalent or excess metal oxidants or organic oxidants (resulting in large amounts of metal and organic waste), high production costs, and a large amount of waste generated in the production process, making the process not green. These processes therefore are still far away from practical application and production.

### SUMMARY

The present disclosure aims to provide a method for preparing a diester by oxidative carbonylation of an olefin and carbon monoxide. The method of the present disclosure has a high catalytic efficiency, a high selectivity, a high conversion rate, and the catalyst is recyclable, and almost no waste is generated during the production process.

The present disclosure provides a method for preparing a diester by oxidative carbonylation of an olefin and carbon monoxide, comprising steps of
under an oxygen-containing atmosphere, performing an oxidative carbonylation reaction of the olefin and carbon monoxide in the presence of a catalyst and a dehydrating agent, to obtain a 1,4-diester-based compound;
wherein the catalyst comprises a main catalyst, an auxiliary oxidant and an acid;
the main catalyst is a substance containing Pd element; the auxiliary oxidant is selected from the group consisting of a copper salt, an iron salt, iron powder and mixtures thereof; the acid is a Lewis acid and/or a protonic acid; and the dehydrating agent is selected from the group consisting of a ketal, an acetal, an orthoformate, a silicate and mixtures thereof.

Preferably, the auxiliary oxidant is selected from the group consisting of a copper salt, an iron salt and a mixture thereof; and the Pd in the main catalyst, the copper salt and the iron salt are in a molar ratio of 1: (0-100): (0-100).

Preferably, the Lewis acid is selected from the group consisting of a boron compound, an aluminum compound, a gallium compound, an indium compound, a zinc compound, a cadmium compound, a mercury compound, a beryllium compound, a magnesium compound, a calcium compound, a strontium compound, a titanium compound, a zirconium compound, a hafnium compound, a scandium compound, a yttrium compound, a lanthanum compound, a praseodymium compound, a neodymium compound, a samarium compound, an europium compound, a gadolinium compound, a terbium compound, a dysprosium compound, a holmium compound, an erbium compound, a thulium compound, a ytterbium compound, a lutetium compound, a germanium compound, a tin compound, an antimony compound, a bismuth compound, and mixtures thereof;
the protonic acid is selected from the group consisting of hydrogen fluoride, hydrogen chloride, hydrogen bromide, hydrogen iodide, sulfuric acid, a sulfonic acid compound, disulfate, selenic acid, telluric acid, nitric acid, phosphoric acid, dihydrogen phosphate, bis(trifluoromethanesulfonyl)imide, fluorosulfonyl imide and mixtures thereof.

Preferably, the acid is selected from the group consisting of BCl₃, BBr₃, AlCl₃, AlBr₃, SnCl₂, SnCl₄, Sc(OTf)₃, Sc(NO₃)₃•H₂O, ScCl₃•6H₂O, Sc₂(SO₄)₃•8H₂O, YCl₃, YBr₃, InCl₃, InBr₃, AuCl₃, PtCl₂, Ce(OTf)₃, TiCl₄, Ti(OR)₄, ClTi(OR)₃, Ti₂(SO₄)₃, ZnCl₂, ZnBr₂, Zn(OTf)₂, HCl, HBr, HI, sulfuric acid, nitric acid, KHSO₄ and mixtures thereof;
the Pd in the main catalyst and the acid are in a molar ratio of 1: (1-2000).

Preferably, the acid is selected from the group consisting of a protonic acid capable of donating H⁺ and a Lewis acid containing a halide ion and a mixture thereof.

Preferably, the Pd in the main catalyst and the olefin are in a molar ratio of 1: (700-1000000); the dehydrating agent and the olefin are in a molar ratio of (0.5-8): 1.

Preferably, the carbon monoxide and the olefin are in a molar ratio of (1-5): 1; and the oxygen in the oxygen-containing atmosphere and the olefin are in a molar ratio of (0.5-3): 1.

Preferably, the oxidative carbonylation reaction is performed at a temperature of 20-300°C under a pressure of 0.5-300 bar for a time of 0.2-40 hours.

Preferably, the oxidative carbonylation reaction is performed in an alkyl alcohol solvent, with the alkyl alcohol solvent and the dehydrating agent being in a molar ratio of 10:1-0.1:10.

Preferably, after the oxidative carbonylation reaction is completed, reaction product is distilled under normal pressure at 170°C, 200°C and 215-220°C, or distilled under reduced pressure, and then residue from the distillation is used as recovered catalyst.

The present disclosure provides a method for preparing a diester by oxidative carbonylation of an olefin and carbon monoxide, comprising steps of: under an oxygen-containing atmosphere, performing an oxidative carbonylation reaction of the olefin and carbon monoxide in the presence of a catalyst, to obtain a 1,4-diester-based compound; wherein the catalyst comprises a main catalyst, an auxiliary oxidant, an acid and a dehydrating agent; the main catalyst is a substance containing Pd element; the auxiliary oxidant is selected from the group consisting of a copper salt, an iron salt, iron powder and mixtures thereof; the acid is a Lewis acid and/or a protonic acid; and the dehydrating agent is selected from the group consisting of a ketal, an acetal, an orthoformate, a silicate and mixtures thereof. The method according to the present disclosure has a high catalytic efficiency (TON> 50000, TOF> 1000/h), a high selectivity (the selectivity of olefin conversion > 99%), and a high conversion rate (the olefin is nearly completely converted). Moreover, the method uses oxygen or air as the oxidant, and the catalyst is recyclable. It is a green, low-carbon and environmentally friendly catalytic system with low cost, and basically no waste is generated during the production process. This system catalyzes the oxidative carbonylation reaction of an olefin with CO to prepare 1,4-diester compounds.

### DETAILED DESCRIPTION

The present disclosure provides a method for preparing a diester by oxidative carbonylation of an olefin and carbon monoxide, comprising the steps of:
under an oxygen-containing atmosphere, performing an oxidative carbonylation reaction of the olefin and carbon monoxide in the presence of a catalyst and a dehydrating agent, to obtain a 1,4-diester-based compound;
wherein the catalyst comprises a main catalyst, an auxiliary oxidant, an acid and a dehydrating agent;
the main catalyst is a substance containing Pd element; the auxiliary oxidant is selected from the group consisting of a copper salt, an iron salt, iron powder and mixtures thereof; the acid is a Lewis acid and/or a protonic acid; and the dehydrating agent is selected from the group consisting of a ketal, an acetal, an orthoformate, a silicate and mixtures thereof.

In the present disclosure, the oxidative carbonylation reaction is shown in Formula I: 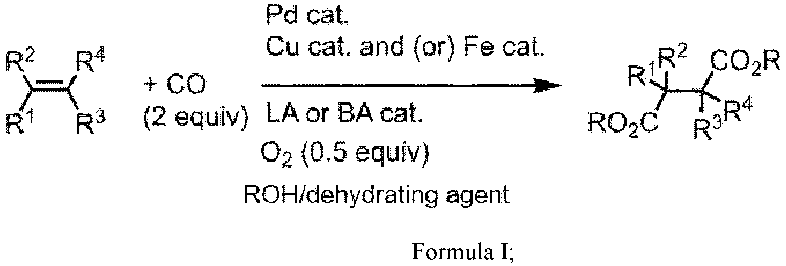 wherein, LA refers to a Lewis acid; BA refers to a Bronsted acid; cat. refers to catalytic amount, and the catalytic amount of each component can be the same or different.

In the present disclosure, the olefin is preferably selected from the group consisting of a substituted or unsubstituted alkylethylene, a substituted or unsubstituted arylethylene, a substituted or unsubstituted cyclic olefin and mixtures thereof, and more preferably from the group consisting of ethylene, propylene, butene, pentene, hexene, styrene, cyclohexene, cyclopentene and mixtures thereof. The molar ratio of the carbon monoxide to the olefin is preferably (1-5): 1, more preferably (2-4): 1, most preferably (2-2.2): 1, for example, 1: 1, 1.5: 1, 2: 1, 2.2: 1, 2.5: 1, 3: 1, 3.5: 1, 4: 1, 4.5: 1, or 5: 1, and is preferably a range value with any of the above values as the upper limit or the lower limit.

In the present disclosure, the catalyst preferably comprises a main catalyst, an auxiliary oxidant and an acid; the main catalyst is preferably a substance containing Pd element, for example, is selected from the group consisting of elemental Pd, a Pd salt, a Pd complex, supported Pd, and mixtures thereof. Specifically, in the embodiments of the present disclosure, the main catalyst may be Pd supported on a carrier such as activated carbon, alumina, SiO₂, molecular sieve or BaSO₄, or can be selected from the group consisting of palladium halide, palladium carboxylate, palladium sulfonate, Pd (dba)₂, Pd(PPh₃)₄ and mixtures thereof. In addition, the valence state of Pd in the main catalyst of the present disclosure can be zero-valent state or divalent state. The molar ratio of the main catalyst to the olefin is preferably 1: (700-1000000), more preferably 1: (1000-100000), most preferably 1: (10000-20000), for example 1: 700, 1: 1000, 1: 5000, 1: 10000, 1: 20000, 1: 50000, 1: 100000, 1: 200000, 1: 500000, or 1: 1000000, and is preferably a range value with any of the above values as the upper limit or the lower limit.

In the present disclosure, the auxiliary oxidant is preferably selected from the group consisting of a copper salt, an iron salt, an iron powder and mixtures thereof, and is preferably a copper salt and/or an iron salt. The copper salt is preferably selected from the group consisting of CuCl, CuBr, CuI, CuOAc, CuCl₂, CuBr₂, Cu(OAc)₂, CuSO₄, Cu(NO₃)₂, Cu(OTf)₂, CuO, basic copper carbonate and mixtures thereof. The iron salt is preferably selected from the group consisting of FeCl₂, FeSO₄, FeCl₃, Fe(NO₃)₃ and the mixtures thereof. Specifically, in the embodiments of the present disclosure, the auxiliary oxidant can be CuCl and/or FeSO₄. The molar ratio of Pd in the main catalyst, the copper salt and the iron salt is preferably 1: (0-100): (0-100), more preferably 1: (1-10): (0.1-5), and most preferably 1: (2-8): (0.2-4).

In the present disclosure, the auxiliary oxidant acts to assist the oxidation of palladium from zero-valent state to divalent state.

In the present disclosure, the acid is preferably a Lewis acid and/or a protonic acid. The Lewis acid is preferably selected from the group consisting of a boron compound, an aluminum compound, a gallium compound, an indium compound, a zinc compound, a cadmium compound, a mercury compound, a beryllium compound, a magnesium compound, a calcium compound, a strontium compound, a titanium compound, a zirconium compound, a hafnium compound, a scandium compound, a yttrium compound, a lanthanum compound, a praseodymium compound, a neodymium compound, a samarium compound, an europium compound, a gadolinium compound, a terbium compound, a dysprosium compound, a holmium compound, an erbium compound, a thulium compound, a ytterbium compound, a lutetium compound, a germanium compound, a tin compound, an antimony compound, a bismuth compound, and mixtures thereof. The compound contained in the Lewis acid can be fluoride, chloride, bromide, iodide, nitrate, sulfate, trifluoromethanesulfonate, bis(trifluoromethane)sulfonimide, bis(fluorosulfonyl)imide, methanesulfonate, benzylsulfonate, p-toluenesulfonate or alkoxide, for example BCl₃, BBr₃, AlCl₃, AlBr₃, SnCl₂, SnCl₄, Sc(OTf)₃, Sc(NO₃)₃•H₂O, ScCl₃•6H₂O, Sc₂(SO₄)₃•8H₂O, YCl₃, YBr₃, InCl₃, InBr₃, AuCl₃, PtCl₂, Ce(OTf)₃, TiCl₄, Ti(OR)₄, ClTi(OR)₃, Ti₂(SO₄)₃, ZnCl₂, ZnBr₂, or Zn(OTf)₂. Preferably, the Lewis acid is a compound containing a halide ion, i.e., chloride. Specifically, in the embodiments of the present disclosure, the Lewis acid can be AlCl₃.

In the present disclosure, the protonic acid (Bronsted acid) is preferably selected from the group consisting of hydrogen fluoride, hydrogen chloride, hydrogen bromide, hydrogen iodide, sulfuric acid, sulfonic acid compounds, disulfate, selenic acid, telluric acid, nitric acid, phosphoric acid, dihydrogen phosphate, bis(trifluoromethanesulfonyl)imide, fluorosulfonyl imide and mixtures thereof, and preferably selected from the group consisting of HCl, sulfuric acid, nitric acid, KHSO₄ and mixtures thereof. In the present disclosure, the protonic acid can also be generated on site through an in-situ preparation method, for example, the reaction of acyl chloride or acyl bromide with methanol can result in hydrogen chloride or hydrogen bromide.

In the present disclosure, the combination of a Lewis acid containing halogen and a protonic acid capable of donating H+ is preferably used, so that the reaction efficiency, especially the efficiency in the process of re cycling the palladium catalyst can be improved. Both the Lewis acid and protonic acid can promote the reaction of the ketal as a dehydrating agent with water, and simultaneously generate 2 equivalents of methanol, that is, the water removal process. The specific process involving protonic acid is shown in Formula II, and the process involving the Lewis acid is similar.

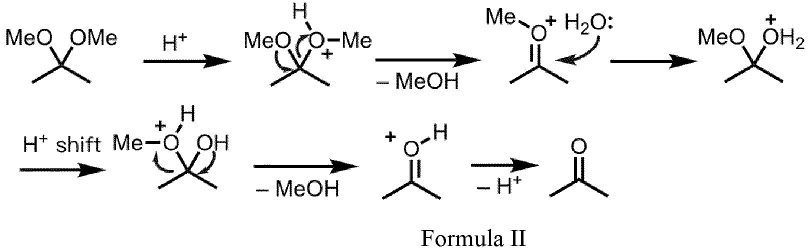

In addition to removing water, the Lewis acid further acts to promote the efficiency of the key carbonyl insertion reaction. It mainly stabilizes the transition state (the process within the box in the following formula), and accelerates the rate at which ligands in the system, such as CO or the solvent, capture the intermediate, thereby speeding up the reaction process. The specific process is shown in Formula III.

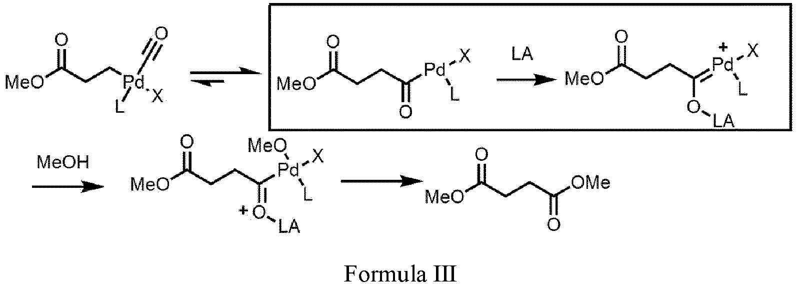

In the present disclosure, the molar ratio of the Lewis acid to the protonic acid is preferably 1: 24-2: 1, more preferably 1:20-1:1, for example, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, and is preferably a range value with any of the above values as the upper limit or the lower limit. The molar ratio of Pd in the main catalyst to the acid is preferably 1: (1-2000), more preferably 1: (100-1200), for example, 1: 1, 1: 10, 1: 20, 1: 30, 1: 40, 1: 50, 1: 60, 1: 70, 1: 80, 1: 90, 1: 100, 1: 200, 1: 300, 1: 400, 1: 500, 1: 600, 1: 700, 1: 800, 1: 900, 1: 1000, 1: 1100, 1: 1200, 1: 1300, 1: 1400, 1: 1500, 1: 1600, 1: 1700, 1: 1800, 1: 1900, 1: 2000, and is preferably a range value with any of the above values as the upper limit or the lower limit.

In the present disclosure, the dehydrating agent is reacted with water to generate acetone, which can be recovered by distillation and then condensed with alcohol to prepare the dehydrating agent, thereby realizing the recycling of the dehydrating agent. The dehydrating agent is selected from the group consisting of a ketal, an acetal, an orthoformate, a silicate and mixtures thereof, and is preferably one or more selected from the group consisting of compounds represented by Formula IV- Formula VII.

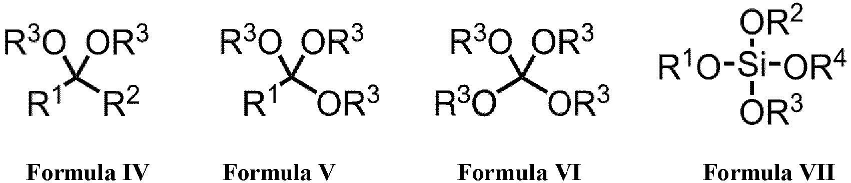

In Formula IV-Formula VII, R¹, R² can independently be hydrogen, alkyl, cycloalkyl, or aryl. R³ is alkyl, cycloalkyl or aryl. R⁴ is alkyl, cycloalkyl or aryl. Specifically, in an embodiment of the present disclosure, the dehydrating agent is 2,2-dimethoxypropane (DMP). The molar ratio of the dehydrating agent to the olefin is preferably (0.5-8): 1, more preferably (1-7): 1, for example, 0.5: 1, 1: 1, 1.5: 1, 2: 1, 2.5: 1, 3: 1, 3.5: 1, 4: 1, 4.5: 1, 5: 1, 5.5: 1, 6: 1, 6.5: 1, 7: 1, 7.5: 1, 8: 1, and is preferably a range value with any of the above values as the upper limit or the lower limit.

In the present disclosure, the oxidative carbonylation reaction is preferably carried out in a reaction solvent. The reaction solvent is preferably an alcohol, more preferably selected from the group consisting of an alkyl alcohol, a cycloalkyl alcohol, an aryl alcohol and mixtures thereof, for example, selected from the group consisting of methanol, ethanol, propanol, isopropanol, n-butanol, isobutanol, tert-butanol, cyclobutanol, n-pentanol, isopentanol, neopentanol, tert-pentanol, cyclopentanol and mixtures thereof, and can also be a phenolic compound. For some substrate compounds that have poor solubility in alcohol solvents, other solvents can be added. The molar ratio of the reaction solvent to the dehydrating agent is preferably 2: 1-1: 3, more preferably 1: 1-1: 2, for example, 2: 1, 1.5: 1, 1: 1, 1: 1.5, 1: 2, 1: 2.5, 1: 3, and is preferably a range value with any of the above values as the upper limit or the lower limit. In the present disclosure, alcohol is both a reactant and a solvent.

In the present disclosure, the oxygen-containing atmosphere can be oxygen, air, or a mixed gas obtained by diluting pure oxygen with nitrogen in a certain proportion. In the oxygen-containing atmosphere, the molar ratio of the oxygen and olefin is preferably (0.5-3): 1, more preferably (1-2.5): 1, for example, 0.5: 1, 1: 1, 1.5: 1, 2: 1, 2.5: 1, 3: 1, and is preferably a range value with any of the above values as the upper limit or the lower limit. In the present disclosure, there is no special restriction on the concentration of oxygen in the oxygen-containing atmosphere, as long as the ratio of oxygen to the olefin is within the range required in the present disclosure.

In the present disclosure, there is no special restriction on the addition sequence of the raw materials. Raw materials can be added together at once according to a set ratio or can be added continuously at a certain flow rate through a gas flow meter. Alternatively, the olefin and carbon monoxide can be added together at once first, and then oxygen can be added continuously through a gas flow meter to keep the oxygen concentration in the reactor within a very safe range.

In the present disclosure, the temperature of the oxidative carbonylation reaction is preferably 20-300°C, more preferably 50-250°C, for example, 20°C, 30°C, 50°C, 100°C, 150°C, 200°C, 250°C, 300°C, and is preferably a range value with any of the above values as the upper limit or the lower limit. The time of the oxidative carbonylation reaction is preferably 0.2-40 hours, more preferably 1-30 hours, for example, 0.2 hour, 1 hour, 5 hours, 10 hours, 15 hours, 20 hours, 25 hours, 30 hours, 35 hours, 40 hours, and is preferably a range value with any of the above values as the upper limit or the lower limit. The pressure of the oxidative carbonylation reaction is 0.5-300 bar, more preferably 10-250 bar, most preferably 50-200 bar, for example, 0.5 bar, 0.8 bar, 1 bar, 10 bar, 30 bar, 50 bar, 100 bar, 150 bar, 200 bar, 250 bar, 300 bar, and is preferably a range value with any of the above values as the upper limit or the lower limit.

The present disclosure provides a method for preparing a diester by oxidative carbonylation of an olefin and carbon monoxide, comprising steps of: under an oxygen-containing atmosphere, performing an oxidative carbonylation reaction of the olefin and carbon monoxide in the presence of a catalyst, to obtain a 1,4-diester-based compound; wherein the catalyst comprises a main catalyst, an auxiliary oxidant, an acid and a dehydrating agent; the main catalyst is a substance containing Pd element; the auxiliary oxidant is selected from the group consisting of a copper salt, an iron salt, iron powder and mixtures thereof; the acid is a Lewis acid and/or a protonic acid; and the dehydrating agent is selected from the group consisting of a ketal, an acetal, an orthoformate, a silicate and mixtures thereof. The method according to the present disclosure has a high catalytic efficiency (TON> 50000, TOF> 1000/h), a high selectivity (the selectivity of olefin conversion > 99%), and a high conversion rate (the olefin is nearly completely converted). Moreover, the method uses oxygen or air as the oxidant, and the catalyst is recyclable. It is a green, low-carbon and environmentally friendly catalytic system with low cost, and basically no waste is generated during the production process. This system catalyzes the oxidative carbonylation reaction of an olefin with CO to prepare 1,4-diester compounds.

To further illustrate the present disclosure, the following examples are further used to provide more details of the method for preparing the diester by oxidative carbonylation of olefin and carbon monoxide provided by the present disclosure, but they should not be interpreted as limitation to the scope of the present invention.

The following examples include abbreviations: 2,2-dimethoxypropane (DMP), dimethyl succinate (DMS), dimethyl oxalate (DMO), dimethyl carbonate (DMC), methyl acrylate (MA).

### Examples

### Example 1. Effect of the type of Pd on the reaction

The specific procedure is introduced using Pd/C as an example. To a 25 mL stainless steel reaction kettle, Pd/C (10% on Carbon, 0.0125 mmol, 13.3 mg), CuCl (0.025 mmol, 2.5 mg), AlCl₃ (0.10 mmol, 13.3 mg), DMP (13.5 mmol, 1660µL), MeOH (dry, 3.4 mL) were added in sequence. The reaction kettle was tightened and sequentially filled with 25 bar of air, 20 bar of CO and 10 bar of ethylene. Then, the reaction kettle was placed in an oil bath with an electromagnetic heating stirrer at 100°C and stirred for 11 h. Other tests were carried out by the same procedure, except that Pd/C was replaced with other different types of palladium.

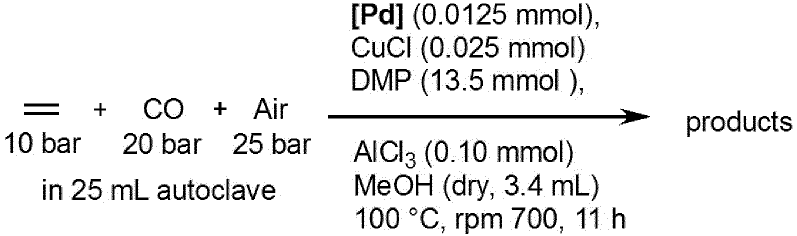

**Table 1. Effect of the type of Pd on the reaction**

| | **[Pd]** | **Yield/mmol** | | |
|---|---|---|---|---|
| | | **DMS** | **DMO** | **DMC** |
| 1 | Pd/Al₂O₃ | 6.59 | 0.10 | 0.19 |
| 2 | Pd/BaSO₄ | 6.45 | 0.12 | 0.24 |
| 3 | Pd/CaCO₃/Pb | 6.40 | 0.19 | 0.44 |
| 4 | Pd/CaCO₃ | 6.26 | 0.10 | 0.28 |
| 5 | Pd/C | 7.05 | 0.19 | 0.30 |
| 6 | PdCl₂ | 7.28 | 0.09 | 0.13 |

The 25 mL stainless steel reaction kettle was used, a total volume of the solution was 5 mL, and mesitylene was used as the internal standard to determine the nuclear magnetic resonance yield. The results are shown in Table 1, indicating that the type of Pd has no significant effect on the reaction activity.

### Example 2. Effect of auxiliary oxidants

The specific procedure is introduced using Cu(acac)₂ as an example. To a 25 mL stainless steel reaction kettle, Pd/C (10% on Carbon, 0.0125 mmol, 13.3 mg), Cu(acac)₂ (0.025 mmol, 6.5 mg), AlCl₃ (0.10 mmol, 13.3 mg), DMP (13.5 mmol, 1660 µL), MeOH (dry, 3.4 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that Cu(acac)₂ was replaced with other different types of Cu.

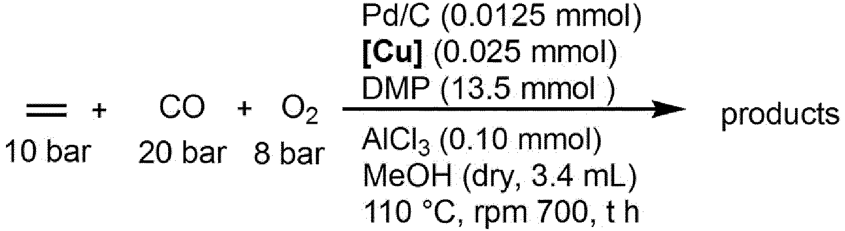

**Table 2. Effect of copper salts**

| | **[Cu]** | **t/h** | **Yield/mmol** | | | | |
|---|---|---|---|---|---|---|---|
| | | | **DMS** | **DMO** | **DMC** | **MA** | **Acetone** |
| 1 | Cu₂(OH)₃Cl 0.0125 mmol | 5 | 7.64 | 0.09 | 0.23 | 0.25 | 7.27 |
| 2 | C₂O₄CU | 11 | 6.58 | 0.04 | 0.08 | -- | 6.30 |
| 3 | Cu(OAc)₂ | 11 | 8.17 | 0.09 | 0.24 | 0.27 | 6.02 |
| 4 | CuI | 11 | 6.94 | 0.04 | 0.15 | 0.07 | 5.36 |
| 5 | Cu(acac)₂ | 11 | 8.51 | 0.10 | 0.42 | 0.16 | 7.06 |
| 6 | CuBr₂ | 11 | 4.01 | 0.09 | 0.10 | -- | 3.61 |
| 7 | CuF₂·2H₂O | 11 | 8.47 | 0.11 | 0.36 | 0.24 | 6.89 |
| 8 | Cu₂O | 11 | 8.64 | 0.12 | 0.40 | 0.41 | 6.94 |
| 9 | **Copper(II) phthalocyanine** | 11 | 6.12 | 0.04 | 0.42 | -- | 7.26 |

The 25 mL stainless steel reaction kettle was used, a total volume of the solution was 5 mL and mesitylene was used as the internal standard to determine the Nuclear Magnetic Resonance yield. The results are shown in Table 2, indicating that copper salts, whether monovalent or divalent, generally can promote the reaction.

The specific procedure is introduced using CuCl₂, AlCl₃, and Fe powder are taken as examples. To a 25 mL of Hastelloy reaction kettle, Pd/C (10% on Carbon, 0.0125 mmol, 13.3 mg), CuCl₂ (0.025 mmol, 3.4 mg), AlCl₃ (0.10 mmol, 13.3 mg), Fe (0.30 mmol, 16.8 mg), DMP (13.5 mmol, 1660 µL), MeOH (dry, 3.4 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that CuCl₂ was replaced with other different types of copper salts, AlCl₃ was replaced with other different types of acids, and Fe powder was replaced with other different types of iron slats.

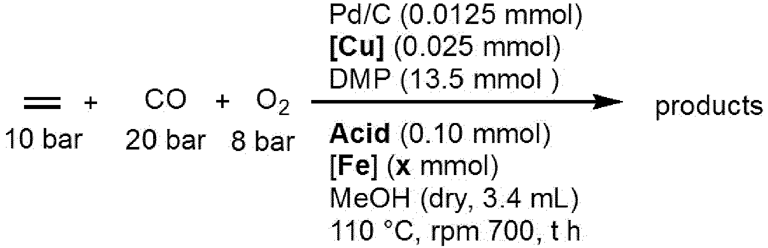

**Table 3. Effect of iron salts**

| | **[Cu]** | **acid** | **[Fe]** | **x** | **t/h** | **Yield/mmol** | | |
|---|---|---|---|---|---|---|---|---|
| | | | | **mmol** | | **DMS** | **DMO** | **DMC** |
| 1 | CuCl | AlCl₃ | -- | -- | 7.5 | 8.50 | 0.27 | 0.70 |
| 2 | CuCl₂ | AlCl₃ | Fe | 0.30 | 12.5 | 8.85 | 0.19 | 0.66 |
| 3 | -- | ACl₃ | Fe | 0.30 | 7.5 | 7.48 | 0.09 | 0.38 |
| 4 | -- | AlCl₃ | Fe | 0.025 | 6 | 6.02 | 0.11 | 0.44 |
| 5 | CuCl | H₂SO₄ | Fe | 0.30 | 13 | 8.12 | 0.28 | 0.55 |
| 6 | CuC1 | H₂SO₄ | -- | -- | 18 | 3.68 | 0.04 | 0.04 |
| 7 | CuCl₂ | H₂SO₄ | -- | -- | 18 | 3.60 | 0.04 | 0.05 |
| 8 | CuCl | H₂SO₄ | FeSO₄ | 0.10 | 12 | 7.42 | 0.06 | 0.07 |
| 9 | CuCl | -- | FeSO₄ | 0.10 | 11.5 | 8.20 | 0.70 | 0.21 |
| 10 | CuSO₄ | H₂SO₄ | Fe | 0.30 | 18 | 1.25 | 1.36 | 1.28 |
| 11 | CuSO₄ | H₂SO₄ | -- | -- | 18 | 0.88 | 0.11 | 0.09 |
| 12 | CuSO₄ | -- | FeSO₄ | 0.10 | 11 | 0.40 | 0.98 | 0.32 |
| 13 | CuCl | -- | Fe | 0.30 | 18 | 0.88 | 1.67 | 1.39 |

The 25 mL of Hastelloy reaction kettle was used, a total volume of the solution was 5 mL, and mesitylene was used as the internal standard to determine the Nuclear Magnetic Resonance yield. The results are shown in Table 3, indicating that iron or iron salts have a certain promoting effect on the reaction and can replace copper salts or be used in combination with copper salts.

### Example 3. Effect of Lewis acids

The specific procedure is introduced using AlCl₃ as an example. To a 25 mL stainless steel reaction kettle, PdCl₂ (0.0125 mmol, 2.2 mg), CuCl (0.025 mmol, 2.5 mg), AlCl₃ (0.025 mmol, 3.3 mg), DMP (13.5 mmol, 1660 µL) and MeOH (dry, 3.4 mL) were added in sequence. The reaction kettle was tightened and sequentially filled with 25 bar of air, 20 bar of CO, and 10 bar of ethylene. Then, the reaction kettle was placed in an oil bath with an electromagnetic heating stirrer at 100°C and stirred. Other tests were carried out by the same procedure, except that AlCl₃ was replaced with other different types of Lewis acid.

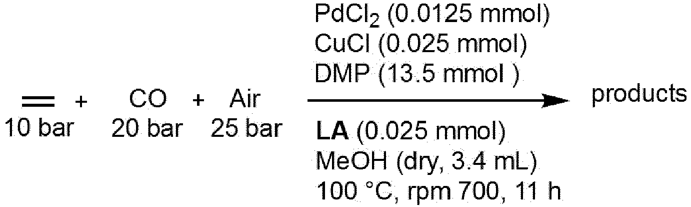

**Table 4. Effect of Lewis acids**

| | **LA** | **Yield/mmol** | | |
|---|---|---|---|---|
| | | **DMS** | **DMO** | **DMC** |
| 1 | Sm(OTf)₃ | 4.86 | 0.40 | 0.29 |
| 2 | Y(OTf)₃ | 4.86 | 0.31 | 0.24 |
| 3 | Mg(OTf)₂ | 3.40 | 0.27 | 0.15 |
| 4 | Yb(OTf)₃ | 4.83 | 0.25 | 0.30 |
| 5 | SnCl₂ | 4.77 | 0.45 | 0.40 |
| 6 | AlCl₃ | 5.34 | 0.24 | 0.22 |
| 7^{[a]} | Ce(OTf)₃ | 5.14 | 0.42 | 0.23 |
| 8^{[a]} | Al(OTf)₃ | 4.39 | 0.51 | 0.25 |
| 9^{[a]} | Lu(OTf)₃ | 5.12 | 0.33 | 0.23 |
| 10^{[a]} | Fe(OTf)₂ | 5.02 | 0.40 | 0.26 |
| 11^{[a]} | Sn(OTf)₂ | 4.23 | 0.76 | 0.28 |
| 12 | Eu(OTf)₃ | 4.90 | 0.37 | 0.22 |
| 13 | Cu(OTf)₂ | 4.02 | 1.08 | 0.69 |
| 14 | Ti(O*ⁿ*Bu)₄ | 5.23 | 0.80 | 0.58 |
| 15 | Ti(O*ⁱ*Pr)₄ | 4.70 | 0.79 | 0.46 |
| 16 | BF₃•Et₂O | 3.26 | 0.50 | 0.34 |
| 17 | Si(OMe)₄ | 3.35 | 0.43 | 0.32 |
| 18^{[b]} | TiF₄ | 4.14 | 0.61 | 0.30 |
| 19 | ClTi(O*ⁱ*Pr)₃ | 5.90 | 0.60 | 0.56 |
| 20 | Ti₂(SO₄)₃ | 5.08 | 0.20 | 0.19 |
| 21 | Ti(OEt)₄ | 5.22 | 0.83 | 0.54 |
| 22^{[a]} | Cp₂TiCl₂ | 5.96 | 0.40 | 0.32 |
| 23^{[a]} | TiCl₄ | 5.90 | 0.23 | 0.30 |
| 24^{[a]} | Sc(OTf)₃ | 6.01 | 0.50 | 0.26 |
| 25^{[c]} | Al(H₂PO₄)₃ | 5.18 | 0.01 | 0.02 |
| 26 | Sc(NO₃)₃·H₂O | 6.41 | 0.09 | 0.12 |
| 27 | ScCl₃·6H₂O | 6.50 | 0.12 | 0.24 |
| 28 | Sc₂(SO₄)₃·8H₂O | 5.24 | 0.17 | 0.11 |
| 29^{[c][d]} | AlCl₃ | 7.05 | 0.19 | 0.30 |
| 30^{[c][d]} | CaCl₂ | 4.15 | 0.55 | 1.07 |

The 25 mL stainless steel reaction kettle was used. The term [a] represents a reaction time of 12 h; the term [b] represents 0.05 mmol of LA; the term [c] represents 0.10 mmol of LA; the term [d] represents the replacement of PdCl₂ with Pd/C. The results are shown in Table 4, indicating that most of the Lewis acids have a promoting effect on the reaction.

### Example 4. Effect of protonic acids

The specific procedure is introduced using KHSO₄ as an example. To a 25 mL stainless steel reaction kettle, Pd/C (10% on Carbon, 0.0125 mmol, 13.3 mg), CuCl (0.025 mmol, 2.5 mg), KHSO₄ (0.30 mmol, 40.8 mg), DMP (13.5 mmol, 1660 µL), MeOH (dry, 3.4 mL) were added in sequence. The reaction kettle was tightened and sequentially filled with 8 bar of O₂, 20 bar of CO, and 10 bar of ethylene. Then, the reaction kettle was placed in an oil bath with an electromagnetic heating stirrer at 110°C and stirred. Other tests were carried out by the same procedure, except that KHSO₄ was replaced with other different types of protonic acids.

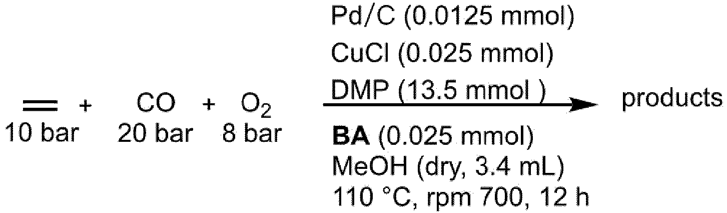

**Table 5. Effect of protonic acids**

| | **BA** | **Yield/mmol** | | |
|---|---|---|---|---|
| | | **DMS** | **DMO** | **DMC** |
| 1^{[a]} | TsOH | 3.68 | 0.36 | 0.30 |
| 2^{[a]} | HCl | 5.48 | 0.29 | 0.30 |
| 3 | H₂SO₄ | 4.14 | 0.03 | 0.22 |
| 4 | HNO₃ (53%) | 5.87 | 0.24 | 0.31 |
| 5^{[b]} | KHSO₄ | 5.96 | 0.72 | 0.91 |
| 6^{[c]} | KHSO₄ | 8.80 | 0.32 | 0.31 |

The 25 mL stainless steel reaction kettle was used, a total volume of the solution was 5 mL, and mesitylene was used as the internal standard to determine the Nuclear Magnetic Resonance yield. The term [a] represents 25 bar of air, a reaction temperature of 100 °C, and a reaction time of 21 h; the term [b] represents 0.15 mmol of **BA;** the term [c] represents 0.30 mmol of **BA.** The results are shown in Table 5, indicating most of the Bronsted acids have a promoting effect on the reaction.

### Example 5. Effect of the proportions of the catalyst components

The specific procedure is introduced using Test 2 in Table 6 as an example. To a 25 mL stainless steel reaction kettle, Pd/C (10% on Carbon, 0.0125 mmol, 13.3 mg), CuCl (0.05 mmol, 5.0 mg), AlCl₃ (0.10 mmol, 13.3 mg), DMP (13.5 mmol, 1660 µL), MeOH (dry, 3.4 mL) were added in sequence. The reaction kettle was tightened and sequentially filled with 25 bar of air, 20 bar of CO, and 10 bar of ethylene. Then, the reaction kettle was placed in an oil bath with an electromagnetic heating stirrer at 100°C and stirred. Other tests were carried out by the same procedure, except that the proportions of CuCl, AlCl₃, DMP were changed.

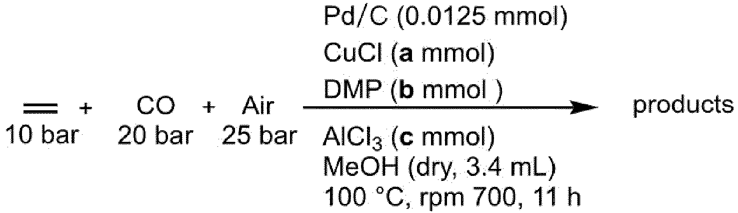

**Table 6. Effect of the proportions of the catalyst components**

| | **mmol** | | | **Yield/mmol** | | |
|---|---|---|---|---|---|---|
| | **a** | **b** | **c** | **DMS** | **DMO** | **DMC** |
| 1^{[c]} | 0.025 | 18 | 0.2 | 6.76 | 0.10 | 0.20 |
| 2 | 0.05 | 13.5 | 0.1 | 6.58 | 0.10 | 0.19 |
| 3^{[c]} | 0.05 | 18 | 0.2 | 6.86 | 0.09 | 0.24 |
| 4^{[a]} | -- | 13.5 | 0.10 | 6.61 | 0.11 | 0.22 |
| 5^{[b]} | -- | 13.5 | 0.10 | 6.14 | 0.07 | 0.18 |
| 6^{[b]} | 0.025 | 13.5 | -- | 5.90 | 0.10 | 0.16 |
| 7 | 0.075 | 13.5 | 0.10 | 7.26 | 0.12 | 0.24 |
| 8^{[a]} | -- | 13.5 | 0.10 | 4.65 | 0.08 | 0.19 |
| 9 | -- | 13.5 | 0.10 | 4.35 | 0.10 | 0.21 |

The 25 mL stainless steel reaction kettle was used, a total volume of the solution was 5 mL, and mesitylene was used as the internal standard to determine the Nuclear Magnetic Resonance yield. The term [a] represents an addition of 0.025 mmol of FeCl₃; the term [b] represents an addition of 0.10 mmol of FeCl₃; the term [c] represents 2.8 mL of MeOH (dry). The results are shown in Table 6.

### Example 6. Effect of dehydrating agents

The specific procedure is introduced using DMP as an example. To a 25 mL stainless steel reaction kettle, Pd/C (10% on Carbon, 0.0125 mmol, 13.3 mg), CuCl (0.025 mmol, 2.5 mg), AlCl₃ (0.10 mmol, 13.3 mg), DMP (13.5 mmol, 1660 µL) and MeOH (dry, 3.4 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that DMP was replaced with other different dehydrating agents.

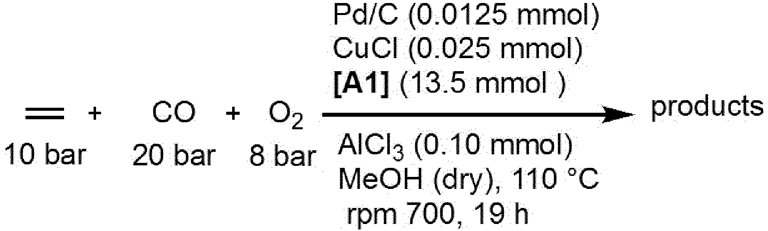

**Table 7. Effect of dehydrating agents**

| | **[A1]** | **Yield/mmol** | | |
|---|---|---|---|---|
| | | **DMS** | **DMO** | **DMC** |
| 1^{[a]} | **DMP** | 8.38 | 0.21 | 0.52 |
| 2 | **CH(OMe)₃** | 8.86 | 0.07 | 0.45 |
| 3 | **Si(OMe)₄** | 8.50 | 0.15 | 0.35 |

The 25 mL stainless steel reaction kettle was used, a total volume of the solution was about 5 mL, and mesitylene was used as the internal standard to determine the Nuclear Magnetic Resonance yield. The term [a] represents a reaction time of 6 h. The results are shown in Table 7, indicating that these organic dehydrating agents in the present disclosure can promote the reaction well.

### Example 7. Effects of proportions of raw materials

The specific procedure is introduced using Test 1 in Table 8 as an example. To a 25 mL stainless steel reaction kettle, Pd/C (10% on Carbon, 0.0125 mmol, 13.3 mg), CuCl (0.025 mmol, 2.5 mg), AlCl₃ (0.10 mmol, 13.3 mg), DMP (13.5 mmol, 1660 µL) and MeOH (dry, 3.4 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that the proportions of the three different gases were changed.

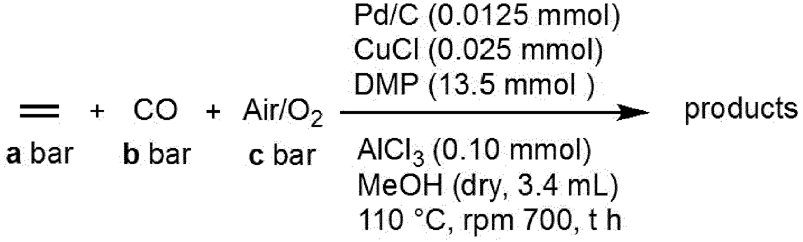

**Table 8. Effect of proportions of raw materials**

| | **bar** | | | **t/h** | **Yield/mmol** | | | |
|---|---|---|---|---|---|---|---|---|
| | **a** | **b** | **c** | | **DMS** | **DMO** | **DMC** | **MA** |
| 1 | 10 | 20 | 8(O₂) | 6 | 8.38 | 0.21 | 0.52 | -- |
| 2 | 10 | 20 | 8(O₂) | 6 | 8.40 | 0.07 | 0.20 | 0.13 |
| 3 | 10 | 20 | 40(Air) | 6 | 10.27 | 0.15 | 0.34 | 0.19 |
| 4 | 10 | 20 | 8(O₂) | 6.5 | 7.90 | 0.12 | 0.36 | 0.13 |
| 5 | 10 | 32 | 8(O₂) | 6.5 | 10.70 | 0.53 | 0.78 | -- |
| 6 | 10 | 20 | 40(Air) | 6.5 | 8.37 | 0.15 | 0.46 | 0.06 |
| 7 | 11 | 20 | 5(O₂) | 6 | 5.56 | 0.06 | 0.23 | -- |
| 8 | 10.5 | 20 | 8(O₂) | 6 | 7.72 | 0.09 | 0.43 | 0.36 |
| 9 | 10 | 20 | 6(O₂) | 6 | 7.49 | 0.05 | 0.19 | -- |
| 10 | 10 | 20 | 7(O₂) | 6 | 8.13 | 0.19 | 0.35 | 0.08 |
| 11 | 10 | 20 | 30(Air) | 6 | 7.37 | 0.13 | 0.29 | -- |
| 12 | 10 | 20 | 6(O₂) | 6.5 | 7.75 | 0.10 | 0.29 | -- |
| 13 | 10 | 20 | 7(O₂) | 6.5 | 8.14 | 0.10 | 0.32 | 0.03 |
| 14 | 11 | 25 | 7(O₂) | 5.5 | 8.39 | 0.15 | 0.32 | -- |
| 15 | 10 | 30 | 7(O₂) | 5.5 | 8.94 | 0.34 | 0.47 | -- |
| 16 | 10 | 32 | 8(O₂) | 5.5 | 7.00 | 0.45 | 0.32 | -- |
| 17 | 10 | 10 | 8(O₂) | 13.5 | 3.16 | 0.04 | 0.32 | 0.51 |
| 18 | -- | 20 | 8(O₂) | 10 | -- | 1.14 | 1.66 | -- |
| 19 | 10 | 30 | 7(O₂) | 15 | 7.89 | 0.03 | 0.41 | -- |

The 25 mL stainless steel reaction kettle was used, a total volume of the solution was 5 mL, and mesitylene was used as the internal standard to determine the Nuclear Magnetic Resonance yield.

### Example 8. Effect of alcohols

The specific procedure is introduced using ethanol as an example. To a 25 mL stainless steel reaction kettle, Pd/C (10% on Carbon, 0.0125 mmol, 13.3 mg), CuCl (0.025 mmol, 2.5 mg), AlCl3 (0.10 mmol, 13.3 mg), [A1] (13.5 mmol, 2176 µL) and EtOH (dry, 2820 µL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that the alcohol and the corresponding ketal were changed. The products are 5.32 mmol of diethyl succinate, 0.1 mmol of diethyl oxalate and 0.08 mmol of diethyl carbonate.

### Example 9. Effects of the proportions of the alcohol and the dehydrating agent

The specific procedure is introduced using Test 2 in Table 9 as an example. To a 25 mL stainless steel reaction kettle, PdCl₂ (0.0125 mmol, 2.2 mg), CuCl (0.025 mmol, 2.5 mg), Sc(OTf)₃ (0.025 mmol, 12.4 mg), DMP (13.5 mmol, 1660 µL) and MeOH (dry, 130.21 mmol, 3.3 mL) were added in sequence. The reaction kettle was tightened and sequentially filled with 25 bar of air, 20 bar of CO and 10 bar of ethylene. Then, the reaction kettle was placed in an oil bath with an electromagnetic heating stirrer at 100°C and stirred. Other tests were carried out by the same procedure, except that the proportions of the alcohol and the dehydrating agent were changed.

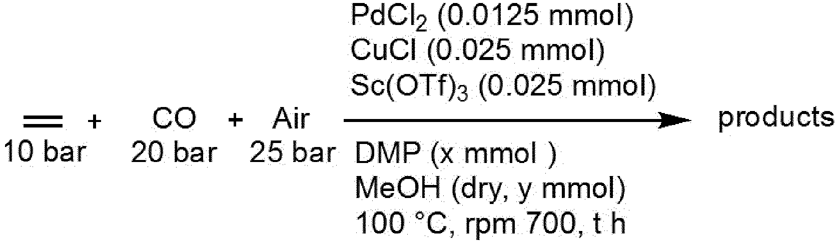

**Table 9. Effects of the proportions of the alcohol and the dehydrating agent**

| | **mmol** | | **t/h** | **Yield/mmol** | | |
|---|---|---|---|---|---|---|
| | **x** | **y** | | **DMS** | **DMO** | **DMC** |
| 1 | 2.25 | 185.45 | 12 | 3.70 | 0.24 | 0.14 |
| 2 | 13.50 | 130.21 | 12 | 6.55 | 0.41 | 0.33 |
| 3 | 48.74 | 18.00 | 12 | 4.57 | 0.06 | 0.08 |
| 4 | 3.375 | 181.50 | 10 | 4.13 | 0.37 | 0.14 |
| 5 | 11.25 | 142.05 | 10 | 5.18 | 0.56 | 0.34 |
| 6 | 13.50 | 134.16 | 10 | 6.13 | 0.61 | 0.32 |
| 7 | 18.00 | 110.48 | 10 | 4.30 | 0.32 | 0.27 |
| 8 | 39.68 | 36.00 | 10 | 4.28 | 0.20 | 0.08 |

The 25 mL stainless steel reaction kettle was used, a total volume of the solution was 5 mL, and mesitylene was used as the internal standard to determine the Nuclear Magnetic Resonance yield.

### Example 10. Effect of other solvents

The specific procedure is introduced using toluene as an example. To a 25 mL stainless steel reaction kettle, Pd/C (10% on Carbon, 0.0125 mmol, 13.3 mg), CuCl (0.025 mmol, 2.5 mg), AlCl3 (0.10 mmol, 13.3 mg), DMP (13.5 mmol, 1660 µL), MeOH (dry, 2.4 mL) and toluene (dry, 1.0 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110°C and stirred. Other tests were carried out by the same procedure, except that toluene was replaced with other solvents.

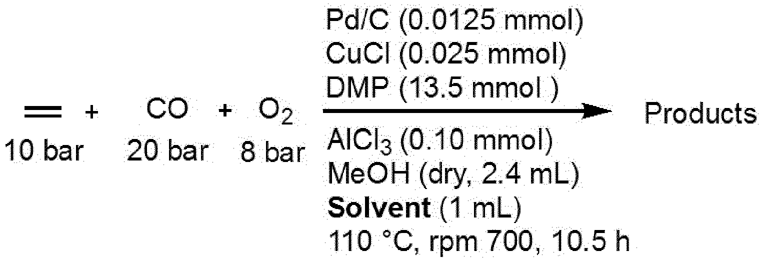

**Table 10. Effect of other solvents**

| **Entry** | **Solvent** | **Yield/mmol** | | | |
|---|---|---|---|---|---|
| | | **DMS** | **DMO** | **DMC** | **MA** |
| 1 | Toluene (dry) | 8.61 | 0.10 | 0.28 | -- |
| 2 | Ethyl acetate | 7.78 | 0.20 | 0.37 | 0.37 |
| 3 | THF (dry) | 8.76 | 0.20 | 0.36 | 0.23 |
| 4 | Acetone (dry) | 7.86 | 0.05 | 0.22 | -- |
| 5 | DCM (dry) | 7.70 | 0.14 | 0.33 | -- |
| 6 | Ether (dry) | 8.82 | 0.11 | 0.26 | 0.33 |

The 25 mL stainless steel reaction kettle was used, a total volume of the solution was 5 mL and mesitylene was used as the internal standard to determine the Nuclear Magnetic Resonance yield. The results are shown in Table 11, indicating that the additional solvent basically has no effect on the reaction efficiency.

### Example 11. Effect of iron powder on the reaction

The specific procedure is introduced using Test 2 in Table 11 as an example. To a 25 mL of Hastelloy reaction kettle, Pd/C (10% on Carbon, 0.0125 mmol, 13.3 mg), CuCl (0.025 mmol, 2.5 mg), AlCl3 (0.10 mmol, 13.3 mg), Fe (0.10 mmol, 5.6 mg), DMP (13.5 mmol, 1660 µL), MeOH (dry, 3.4 mL) were added in sequence. The reaction kettle was tightened and sequentially filled with 25 bar of air, 20 bar of CO and 10 bar of ethylene. Then, the reaction kettle was placed in an oil bath with an electromagnetic heating stirrer at 100 °C and stirred. Other tests were carried out by the same procedure, except that the amount of iron powder was changed.

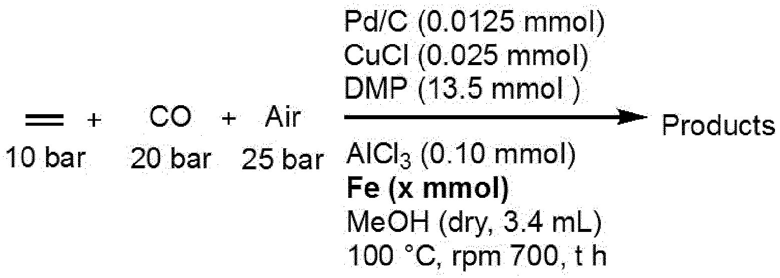

**Table 11. Effect of iron powder**

| | **x/mmol** | **t/h** | **Yield/mmol** | | |
|---|---|---|---|---|---|
| | | | **DMS** | **DMO** | **DMC** |
| 1 | -- | 17 | 7.22 | 0.25 | 0.46 |
| 2 | 0.10 | 10 | 6.84 | 0.32 | 0.65 |
| 3 | 0.20 | 10 | 6.22 | 0.20 | 0.36 |
| 4 | 0.30 | 11 | 7.20 | 0.21 | 0.48 |
| 5 | 0.40 | 11 | 7.20 | 0.28 | 0.48 |
| 6 | 0.50 | 9 | 6.97 | 0.37 | 0.56 |
| 7 | 1.00 | 10 | 3.82 | 0.62 | 0.61 |

The 25 mL of Hastelloy reaction kettle was used, a total volume of the solution was 5 mL and mesitylene was used as the internal standard to determine the Nuclear Magnetic Resonance yield. The iron powder was converted into Fe salt.

### Example 12. Effect of reaction parameters

The specific procedure is introduced using Test 1 in Table 12 as an example. To a 25 mL of Hastelloy reaction kettle, Pd/C (10% on Carbon, 0.0125 mmol, 13.3 mg), CuCl (0.025 mmol, 2.5 mg), AlCl3 (0.10 mmol, 13.3 mg), Fe (0.30 mmol, 16.8 mg), DMP (13.5 mmol, 1660 µL), and MeOH (dry, 3.4 mL) were added in sequence. The reaction kettle was tightened and sequentially filled with 25 bar of air, 20 bar of CO and 10 bar of ethylene. Then, the reaction kettle was placed in an oil bath with an electromagnetic heating stirrer at 100 °C and stirred. Other tests were carried out by the same procedure, except that the pressure of the filled air, the reaction temperature and the reaction time were changed.

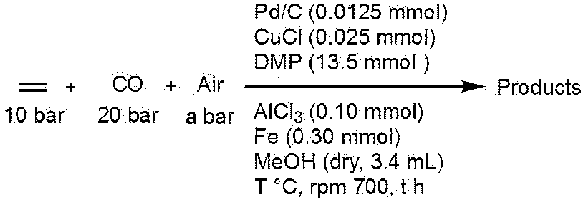

**Table 12. Effect of reaction parameters**

| | **a/bar** | **T/°C** | **t/h** | **Yield/mmol** | | | |
|---|---|---|---|---|---|---|---|
| | | | | **DMS** | **DMO** | **DMC** | **MA** |
| 1 | 25 | 100 | 11 | 7.20 | 0.21 | 0.48 | -- |
| 2 | 25 | 110 | 4.5 | 6.78 | 0.24 | 0.49 | -- |
| 3 | 25 | 120 | 3.5 | 6.79 | 0.21 | 0.59 | -- |
| 4 | 30 | 130 | 1.5 | 10.10 | 0.12 | 0.66 | 0.06 |
| 5 | 30 | 140 | 1.5 | 8.10 | 0.10 | 0.66 | 0.07 |
| 6 | 30 | 150 | 40 min | 7.75 | 0.07 | 0.58 | 0.22 |
| 7 | 30 | 160 | 40 min | 8.66 | 0.06 | 0.66 | 0.13 |

The 25 mL of Hastelloy reaction kettle was used, a total volume of the solution was 5 mL and mesitylene was used as the internal standard to determine the Nuclear Magnetic Resonance yield.

### Comparative Examples

The specific procedure is introduced using Test 11 in Table 13 as an example. To a 25 mL of Hastelloy reaction kettle Pd/C (10% on Carbon, 0.0125 mmol, 13.3 mg), CuCl₂ (0.025 mmol, 3.4 mg), AlCl3 (0.10 mmol, 13.3 mg), Fe (0.30 mmol, 16.8 mg), DMP (13.5 mmol, 1660 µL) and MeOH (dry, 3.4 mL) were added in sequence. The reaction kettle was tightened and sequentially filled with 8 bar of O₂, 20 bar of CO and 10 bar of ethylene. Then, the reaction kettle was placed in an oil bath with an electromagnetic heating stirrer at 110 °C and stirred. Other tests were carried out by the same procedure, except that the relevant variables were increased or decreased.

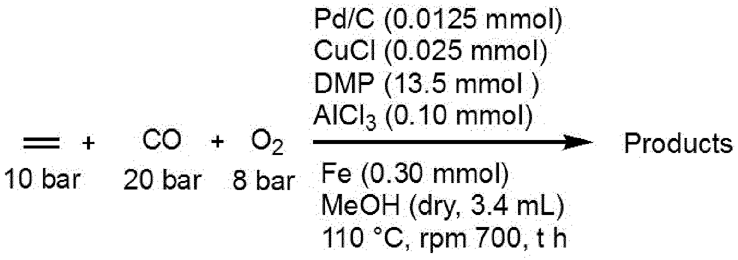

**Table 13. Reaction efficiency of the comparative example**

| | Conditions | **t/h** | **Yield/mmol** | | |
|---|---|---|---|---|---|
| | | | **DMS** | **DMO** | **DMC** |
| 1 | no Pd/C | 16 | 0.12 | 0.28 | 1.46 |
| 2 | no Pd/C and CuCl | 16 | 0.05 | 0.003 | 0.005 |
| 3 | no Fe | 7.5 | 8.50 | 0.27 | 0.70 |
| 4 | no CuCl | 7.5 | 7.48 | 0.09 | 0.38 |
| 5 | no CuCl | 12.5 | 8.44 | 0.08 | 0.42 |
| 6 | no CuCl | 11 | 7.83 | 0.06 | 0.42 |
| 7 | no CuCl and Fe | 11 | 3.85 | 0.09 | 0.23 |
| 8^{[a]} | no AlCl₃ and Fe | 16 | 2.87 | 2.61 | 1.75 |
| 9^{[a]} | no DMP and Fe | 16 | 2.99 | 0.01 | 0.07 |
| 10^{[a]} | no MeOH and Fe | 16 | 7.60 | 0.03 | 0.04 |
| 11^{[b]} | - | 12.5 | 8.85 | 0.19 | 0.66 |

The 25 mL of Hastelloy reaction kettle was used, a total volume of the solution was 5 mL, and mesitylene was used as the internal standard to determine the Nuclear Magnetic Resonance yield. The term [a] represents a 25 mL stainless steel reaction kettle, and the term [b] represents the replacement of CuCl with CuCl₂ (0.025 mmol). The results are shown in Table 13, indicating that Pd is essential, at least one of Fe and Cu is required, and adding both of them can improve the efficiency, and the dehydrating agent and the Lewis acid can significantly improve the reaction efficiency.

### Optimization test

The specific procedure is introduced using Test 1 in Table 14 as an example. To an 80 mL stainless steel reaction kettle, Pd/C (10% on Carbon, 0.0125 mmol, 13.3 mg), CuCl (0.075 mmol, 7.4 mg), AlCl3 (0.30 mmol, 40.0 mg), DMP (100 mmol, 12296 µL) and MeOH (dry, 10.0 mL) were added in sequence. The reaction kettle was tightened and sequentially filled with 40 bar of CO, 20 bar of ethylene and 15 bar of O₂. Then, the reaction kettle was placed in an oil bath with an electromagnetic heating stirrer at 110°C and stirred. Other tests were carried out by the same procedure, except that the proportions of CO and O₂, the amounts of Pd/C, CuCl and AlCl₃, the reaction temperature and the reaction time were changed.

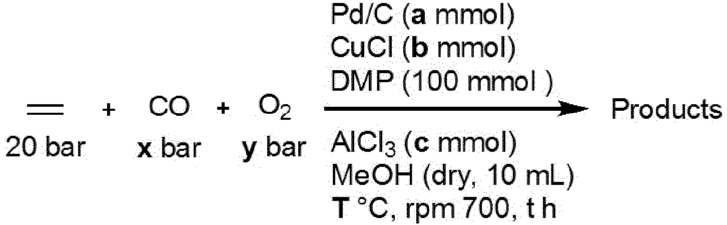

**Table 14. Optimization of the reaction efficiency**

| | **x** | **y** | **a** | **b** | **c** | T/°C | t/h | TON | **Yield/mmol** | | | | |
|---|---|---|---|---|---|---|---|---|---|---|---|---|---|
| | bar | | mmol | | | | | | DMS | DMO | DMC | MA | |
| 1 | 40 | 15 | 0.0125 | 0.075 | 0.30 | 110 | 11 | 4234 | 52.92 | 1.73 | 1.43 | -- | |
| 2 | 40 | 20 | 0.005 | 0.075 | 0.3 | 120 | 13 | 8464 | 42.32 | 1.70 | 2.45 | -- | |
| 3 | 40 | 20 | 0.005 | 0.03 | 0.12 | 120 | 15 | 11896 | 59.48 | 2.75 | 2.15 | 1.10 | |
| 4 | 45 | 15 | 0.005 | 0.03 | 0.12 | 120 | 20 | 11536 | 57.68 | 3.20 | 1.55 | -- | |
| 5 | 45 | 20 | 0.003 | 0.018 | 0.12 | 130 | 20 | 20675 | 62.02 | 3.53 | 2.52 | -- | |
| 6 | 40 | 20 | 0.001 | 0.006 | 0.12 | 130 | 15 | 28150 | 28.15 | 2.98 | 1.68 | -- | |
| 7 | 45 | 20 | 0.001 | 0.006 | 0.24 | 130 | 35 | 40475 | 40.48 | 1.78 | 2.45 | -- | |
| 8 | 45 | 20 | 0.001 | 0.006 | 0.48 | 130 | 30 | 39650 | 39.65 | 0.95 | 1.92 | -- | |
| 9 | 45 | 20 | 0.005 | 0.015 | 0.12 | 120 | 20 | 11640 | 58.20 | 2.62 | 2.05 | 0.40 | |
| 10 | 45 | 20 | 0.005 | 0.010 | 0.12 | 120 | 20 | 12200 | 61.00 | 2.53 | | 1.35 | -- |

The 80 mL stainless steel reaction kettle was used, a total volume of the reaction solution was 22 mL and mesitylene was used as the internal standard to determine the Nuclear Magnetic Resonance yield. The results are shown in Table 14. When about 60 mmol of product was produced in the reaction, the ethylene in the reaction kettle was nearly completely reacted.

### Screening and optimization of conditions of a 400 mL reaction kettle:

The specific procedure is introduced using Test 1 in Table 15 as an example. To a 400 mL reaction kettle (with polytetrafluoroethylene lining), Pd/C (10% on Carbon, 0.001 mmol, 1.1 mg), CuCl (0.006 mmol, 0.6 mg), FeCl₂(0.30 mmol, 38.5 mg), AlCl3 (1.00 mmol, 133.3 mg), DMP (500 mmol, 61481.8 µL) and MeOH (dry, 40.0 mL) were added in sequence. The reaction kettle was tightened and sequentially filled with 20 bar of CO, 20 bar of ethylene, 25 bar of CO and 20 bar of O₂. Then, the reaction kettle was placed in an electromagnetic heating stirrer at 130°C and stirred for 20 h. After being cooled to room temperature by ice bath, the reaction kettle was depressurized to normal pressure in a fume hood to release the unreacted gas, the reaction kettle was opened to add mesitylene (10 mmol, 1395 µL) as the internal standard, and stirred for 2 min. To a 1.5 mL microcentrifuge tube, 0.7 mL CDCl₃ and 10 µL of reaction solution were added, shaken for 30 s and centrifuged for 30 s, and then the obtained products were determined by ¹H NMR. The yield of each product was determined by comparing the proton integrals of these products with those of the internal standard mesitylene. Other tests were carried out by the same procedure, except that the proportions of various variables were changed, the corresponding variables were increased or decreased, and the proportions of different gases and the reaction times were changed.

**Table 15. Screening and optimization of conditions of the 400 ml reaction kettle**

| **Entry** | **mmol** | | | | **t/h** | **TON** | **Yield/mmol** | | | |
|---|---|---|---|---|---|---|---|---|---|---|
| | **a** | **b** | **c** | **d** | | | **DMS** | **DMO** | **DMC** | **MA** |
| 1 | 0.001 | 0.006 | 1.0 | 0.30 | 20 | 52970 | 52.97 | 10.18 | 6.05 | -- |
| 2 | -- | 0.006 | 2.0 | 0.30 | 20 | -- | 1.15 | 3.15 | 9.02 | -- |
| 3 | 0.005 | 0.03 | 2.00 | 0.30 | 22 | 38004 | 190.02 | 9.45 | 11.98 | -- |
| 4^{[a]} | 0.005 | 0.03 | 2.00 | 0.30 | 48 | 31910 | 159.55 | 7.80 | 9.58 | -- |
| 5^{[b]} | 0.005 | 0.03 | 2.30 | -- | 21 | 17476 | 87.38 | 9.90 | 7.00 | -- |
| 6 | 0.005 | 0.015 | 2.00 | 0.30 | 23 | 39576 | 197.88 | 8.70 | 11.08 | -- |
| 7 | 0.005 | -- | 2.00 | 0.30 | 26 | 35104 | 175.52 | 10.47 | 11.67 | -- |
| 8 | 0.01 | 0.03 | 2.00 | 0.30 | 22 | 20972 | 209.72 | 10.07 | 11.48 | -- |
| 9 | 0.01 | 0.06 | 2.00 | 0.10 | 27.5 | 22642 | 226.42 | 11.23 | 10.87 | -- |
| 10^{[c][d]} | 0.01 | 0.06 | 2.00 | 0.10 | 28 | 22605 | 226.05 | 15.50 | 12.05 | -- |
| 11^{[d]} | 0.02 | 0.12 | 2.00 | 0.10 | 21.5 | 11506 | 230.12 | 16.72 | 12.07 | -- |
| 12^{[d][e]} | 0.01 | 0.06 | 2.00 | 0.10 | 28.5 | 22018 | 220.18 | 18.90 | 14.83 | -- |

The 400 mL reaction kettle (with polytetrafluoroethylene lining) was used, a total volume of the solution was about 100 mL and mesitylene (10 mmol) was used as the internal standard.

The term [a] represents a reaction temperature of 120°C; the term [b] represents no FeCl₂; the term [c] represents replacement of Pd/C with PdCl₂; and the term [d] represents 50 bar of CO; [e] 600 mmol of DMP, 26 mL of MeOH and 25 bar of O₂.

Stepwise oxygenation reaction in a 400 mL reaction kettle (with polytetrafluoroethylene lining):
The specific procedure is introduced using Test 1 in Table 16 as an example. To a 400 mL reaction kettle (with polytetrafluoroethylene lining), Pd/C (10% on Carbon, 0.02 mmol, 21.3 mg), CuCl (0.12 mmol, 11.9 mg), FeCl₂ (0.10 mmol, 12.7 mg), AlCl3 (2.00 mmol, 266.7 mg), DMP (600 mmol, 73778.0 µL) and MeOH (dry, 26.0 mL) were added in sequence. The reaction kettle was tightened and sequentially filled with 20 bar of CO, 20 bar of ethylene, 30 bar of CO and 15 bar of O₂. Then, the reaction kettle was placed on an electromagnetic heating stirrer at 130°C and stirred for 10 h to complete the first stage reaction. Subsequently, after being cooled to room temperature by an ice bath, the reaction kettle was filled with 10 bar of O₂. Then, the reaction kettle was placed in an electromagnetic heating stirrer at 130°C and stirred for 15 h to complete the second stage reaction. After being cooled to room temperature by ice bath, the reaction kettle was depressurized to normal pressure in a fume hood to release the unreacted gas, the reaction kettle was opened to add mesitylene as the internal standard (10 mmol, 1395 µL) and stirred for 2 min. To a 1.5 mL microcentrifuge tube, 0.7 mL CDCl₃ and 10 µL of reaction solution were added, shaken for 30 s, and centrifuged for 30 s, and then the obtained products were determined by ¹H NMR. The yield of each product was determined by comparing the proton integrals of these products with those of the internal standard mesitylene. Other tests were carried out by the same procedure, except that the proportion of the oxygen, the proportions of DMP and methanol and the reaction time were changed.

**Table 16. Stepwise oxygenation reaction in the 400 ml reaction kettle (with polytetrafluoroethylene lining)**

| **Entry** | **a** | **b** | **c** | **t/h** | **TON** | **Yield/mmol** | | | |
|---|---|---|---|---|---|---|---|---|---|
| | **bar** | **mmol** | **mL** | | | **DMS** | **DMO** | **DMC** | **MA** |
| 1^{[a]} | 10+14 | 600 | 26 | 10+36 | 25230 | 252.30 | 21.82 | 15.28 | -- |
| 2 | 10+10 | 500 | 40 | 10.5+24 | 11895 | 237.90 | 16.27 | 11.78 | -- |
| 3 | 15+10 | 500 | 40 | 10+11 | 11830 | 236.60 | 16.63 | 14.10 | -- |

The 400 mL reaction kettle (with polytetrafluoroethylene lining) was used, a total volume of the solution was about 100 mL and mesitylene (10 mmol) was used as the internal standard. The term [a] represents 0.01 mmol of Pd/C and 0.06 mmol of CuCl.

### Catalyst recovery experiment in a 400mL reaction kettle:

Procedure for the first reaction experiment: To a 400 mL reaction kettle (with polytetrafluoroethylene lining), Pd/C (10% on Carbon, 0.02 mmol, 21.3 mg), CuCl (0.12 mmol, 11.9 mg), FeCl₂ (0.10 mmol, 12.7 mg), AlCl3 (2.00 mmol, 266.7 mg), DMP (600 mmol, 73778.0 µL) and MeOH (dry, 26.0 mL) were added in sequence. The reaction kettle was tightened and sequentially filled with 20 bar of CO, 20 bar of ethylene, 30 bar of CO and 15 bar of O₂. Then, the reaction kettle was placed in an electromagnetic heating stirrer at 130°C and stirred for 13 h to complete the first stage reaction. Subsequently, after being cooled to room temperature by an ice bath, the reaction kettle was filled with 10 bar of O₂. Then, the reaction kettle was placed in an electromagnetic heating stirrer at 130°C and stirred for 26.5 h, to complete the second stage reaction. After being cooled to room temperature by ice bath, the reaction kettle was depressurized to normal pressure in a fume hood to release the unreacted gas, the reaction kettle was opened to add mesitylene as the internal standard (10 mmol, 1395 µL), and stirred for 2 min. To a 1.5 mL microcentrifuge tube, 0.7 mL CDCl₃ and 10 µL of reaction solution were added, shaken for 30 s and centrifuged for 30 s, and then the obtained products were determined by ¹H NMR. The yield of each product was determined by comparing the proton integrals of these products with those of the internal standard mesitylene. Subsequently, the reaction solution was transferred to a 250 mL round-bottom flask, distilled at 170°C and 200°C under normal pressure to separate the preceding fractions (mainly including methanol, acetone, DMO, DMC, ME, ODS, etc.), and then distilled at 215-220°C under normal pressure to separate the product DMS. Finally, the residue in the round-bottom flask was transferred into a 400 mL reaction kettle (with polytetrafluoroethylene lining) as the catalyst for the recovery experiment.

Procedure for the recovery experiment: The procedure is introduced using the second recovery reaction as an example. To a 400 mL reaction kettle (with polytetrafluoroethylene lining), the recovered catalyst (the residue obtained from the previous distillation of the reaction solution), DMP (600 mmol, 73778.0 µL), MeOH (dry, 26.0 mL) and HCl (4 mol/L in MeOH, 12 mmol, 3.0 mL) were added in sequence. The reaction kettle was tightened and sequentially filled with 20 bar of CO, 20 bar of ethylene, 30 bar of CO and 15 bar of O₂. Then, the reaction kettle was placed in an electromagnetic heating stirrer at 130°C and stirred for 15 h to complete the first stage reaction. Subsequently, after being cooled to room temperature by an ice bath, the reaction kettle was filled with 10 bar of O₂, then placed in an electromagnetic heating stirrer at 130°C and stirred for 7 h, to complete the second stage reaction. After being cooled to room temperature by ice bath, the reaction kettle was depressurized to normal pressure in a fume hood to release the unreacted gas, the reaction kettle was opened to add CH₂Br₂ as the internal standard (10 mmol, 700 µL) and stirred for 2 min. To a 1.5 mL microcentrifuge tube, 0.7 mL CDCl₃ and 10 µL of reaction solution were added, shaken for 30 s, and centrifuged for 30 s, and then the obtained products were determined by ¹H NMR. The yield of each product was determined by comparing the proton integrals of these products with those of the internal standard CH₂Br₂. Subsequently, the reaction solution was transferred to a 250 mL round-bottom flask, distilled at 170°C and 200°C under normal pressure to separate the preceding fractions (mainly including methanol, acetone, DMO, DMC, ME, ODS, etc.), and then distilled at 215-220°C under normal pressure to separate the product DMS. Finally, the residue in the round-bottom flask was transferred into a 400 mL reaction kettle (with polytetrafluoroethylene lining) as the catalyst for the recovery experiment. Other recovery experiments were carried out by the same procedure as this experiment, except that the reaction time of the first and second stages were changed and the relevant variables were increased and decreased.

**Table 17. Catalyst recovery experiments**

| **Entry** | **Recycling times** | **t/h** | **TON** | **NMR yields/mmol** | | | **Distillation yields** |
|---|---|---|---|---|---|---|---|
| | | | | **DMS** | **DMO** | **DMC** | **DMS (g)** |
| 1^{[a]} | 0 | 13+26.5 | 12300 | 246.00 | 18.42 | 14.03 | 31.72 |
| 2^{[b]} | 1 | 21 | 427.5 | 8.55 | 6.60 | 4.05 | -- |
| 3 | 2 | 15+7 | 11751 | 235.03 | 19.13 | 15.53 | 32.03 |
| 4 | 3 | 9.5+11 | 11851 | 237.02 | 23.65 | 20.33 | 30.75 |
| 5 | 4 | 14+26 | 13418 | 268.37 | 25.15 | 16.50 | 36.68 |

| | | | | | | | |
|---|---|---|---|---|---|---|---|
| Note: The 400 mL reaction kettle was used, a total volume of the solution was about 100 mL and dibromomethane (10 mmol) was used as the internal standard. | | | | | | | |

The catalyst for the recovery experiment was the residue after distillation of the previous reaction solution, which was then transferred to the reaction kettle.

The term [a] represents using mesitylene (10 mmol) as the internal standard.

The term [b] represents no addition of HCl, 26 mL MeOH (dry), only 15 bar of O₂ filled, almost no change of gas pressure after 21 hours.

Experiment of Entry 2 demonstrated that the reaction requires not only aluminum (being as Lewis acid, AlCl3 will react with MeOH during the distillation process to lose HCl and obtain Al(OMe)₃), but also a protonic acid.

### Substrate expansion

The specific procedure is introduced using 4-methylstyrene as an example. To a 25 mL Hastelloy reaction kettle, Pd/C (10% on Carbon, 0.01 mmol, 10.6 mg), CuCl (0.02 mmol, 2.0 mg), AlCl3 (0.10 mmol, 13.3 mg), 4-methylstyrene (10 mmol, 1317.4 µL), DMP (20 mmol, 2459.3 µL), and MeOH (dry, 2.6 mL) were added in sequence. The reaction kettle was tightened and sequentially filled with 16 bar of O₂ and 40 bar of CO. Then, the reaction kettle was placed in an oil bath at 120°C with an electromagnetic heating stirrer and stirred for 24 h. After being cooled to room temperature by ice bath, the reaction kettle was depressurized to normal pressure in a fume hood to release the unreacted gas, the reaction kettle was opened to add an internal standard CH₂Br₂ (1 mmol, 70.0 µL), and stirred for 2 min. To a 1.5 mL microcentrifuge tube, 0.7 mL CDCl₃ and 10 µL of reaction solution were added, shaken for 30 s, and centrifuged for 30 s, and then the obtained products were determined by ¹H NMR. The yield of each product was determined by comparing the proton integrals of these products with those of the internal standard CH₂Br₂.

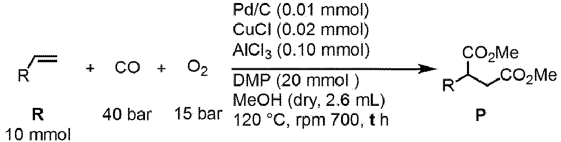

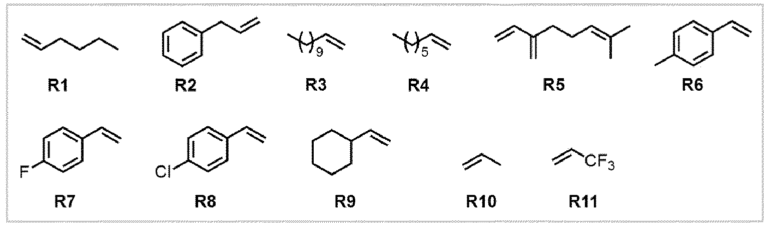

Other experiments for substrate expansion were carried out by the above standard procedure, except that different reaction conditions including different olefins, different component ratios, different gas ratios, different reaction temperatures, and different reaction times were used.

**Table 18. Yields of different substrates**

| Entry | R | Amounts of raw material R recovered/mmol | t/h | Yield/mmol |
|---|---|---|---|---|
| | | | | P1 |
| 1^{[a]} | R1 | 0.23 | 11 | 7.91 |
| 2 | R2 | 0 | 15 | 9.44 |
| 3 | R3 | 0 | 15 | 9.28 |
| 4 | R4 | 0 | 15 | 8.81 |
| 5 | R5 | 1.34 | 40.5 | 3.61 |
| 6 | R6 | -- | 16. | 9.67 |
| 7 | R7 | 0.12 | 20 | 10.27 |
| 8 | R8 | 0.07 | 20 | 9.25 |
| 9 | R9 | 0.37 | 16.5 | 5.90 |
| 10^{[b]} | R10 | -- | 20 | 33.90 |
| 11^{[c]} | R11 | | 20 | 4.70 |

These experiments were carried out in a 25 mL Hastelloy reaction kettle and using dibromomethane (1 mmol) as an internal standard. The term [a] represents replacement of Pd/C with 0.01 mmol PdCl₂, addition of 13 mmol DMP, 3.4 mL MeOH (dry), 22 bar of CO, 8 bar of O₂ at 110°C, and use of mesitylene (1 mmol) as the internal standard. The term [b] represents an 80 mL stainless steel reaction kettle, 0.005 mmol of Pd/C, 0.03 mmol of CuCl, 0.30 mmol of FeCl₂, 0.25 mmol of AlCl₃, 100 mmol of DMP, 10.0 mL of MeOH (dry), 10 bar of propylene, 25 bar of CO, 15 bar of O₂. The term [c] represents an 80 mL stainless steel reaction kettle, 0.01 mmol of Pd/C, 0.06 mmol of CuCl, 0.50 mmol of AlCl₃, 100 mmol of DMP, 10.0 mL of MeOH (dry), about 70 mmol of 3,3,3-trifluoropropene, 50 bar of CO, 20 bar of O₂ at 130°C.

As can be seen from these examples, the catalyst combination system in the present disclosure has a particularly high compatibility with the main catalyst Pd. Whether it is elemental Pd (including supported Pd) or high-valence Pd, similar results can be obtained. The effect obtained by the catalytic system in the present disclosure far exceeds the data reported in the published patents and papers (the highest conversion number does not exceed 300), whereas the highest conversion number according to the methods in the present disclosure exceeds 50000. The catalyst for the reaction can be recovered, the recovery is simple, and there is no impact on the catalytic activity. It is suitable for commercialization.

The above description is only the preferred embodiments of the present disclosure. It should be noted that those skilled in the art may make various improvements and modifications without departing from the principle of the present invention, and these improvements and modifications should fall within the scope of protection of the present invention.

## Claims

1. A method for preparing a diester by oxidative carbonylation of an olefin and carbon monoxide, comprising:
under an oxygen-containing atmosphere, performing an oxidative carbonylation reaction of the olefin and carbon monoxide in the presence of a catalyst and a dehydrating agent, to obtain a 1,4-diester-based compound;
wherein the catalyst comprises a main catalyst, an auxiliary oxidant and an acid;
the main catalyst is a substance containing Pd element; the auxiliary oxidant is selected from the group consisting of a copper salt, an iron salt, iron powder and mixtures thereof; the acid is a Lewis acid and/or a protonic acid; and the dehydrating agent is selected from the group consisting of a ketal, an acetal, an orthoformate, a silicate and mixtures thereof.

2. The method according to claim 1, wherein the auxiliary oxidant is selected from the group consisting of a copper salt, an iron salt and a mixture thereof; and the Pd in the main catalyst, the copper salt and the iron salt are in a molar ratio of 1: (0-100): (0-100).

3. The method according to claim 1, wherein the Lewis acid is selected from the group consisting of a boron compound, an aluminum compound, a gallium compound, an indium compound, a zinc compound, a cadmium compound, a mercury compound, a beryllium compound, a magnesium compound, a calcium compound, a strontium compound, a titanium compound, a zirconium compound, a hafnium compound, a scandium compound, a yttrium compound, a lanthanum compound, a praseodymium compound, a neodymium compound, a samarium compound, an europium compound, a gadolinium compound, a terbium compound, a dysprosium compound, a holmium compound, an erbium compound, a thulium compound, a ytterbium compound, a lutetium compound, a germanium compound, a tin compound, an antimony compound, a bismuth compound and mixtures thereof;
the protonic acid is selected from the group consisting of hydrogen fluoride, hydrogen chloride, hydrogen bromide, hydrogen iodide, sulfuric acid, a sulfonic acid compound, disulfate, selenic acid, telluric acid, nitric acid, phosphoric acid, dihydrogen phosphate, bis(trifluoromethanesulfonyl)imide, fluorosulfonyl imide and mixtures thereof.

4. The method according to claim 3, wherein the acid is selected from the group consisting of BCl₃, BBr₃, AlCl₃, AlBr₃, SnCl₂, SnCl₄, Sc(OTf)₃, Sc(NO₃)₃•H₂O, ScCl₃•6H₂O, Sc₂(SO₄)₃•8H₂O, YCl₃, YBr₃, InCl₃, InBr₃, AuCl₃, PtCl₂, Ce(OTf)₃, TiCl₄, Ti(OR)₄, ClTi(OR)₃, Ti₂(SO₄)₃, ZnCl₂, ZnBr₂, Zn(OTf)₂, HCl, HBr, HI, sulfuric acid, nitric acid, KHSO₄ and mixtures thereof; and
the Pd in the main catalyst and the acid are in a molar ratio of 1: (1-2000).

5. The method according to claim 4, wherein the acid is selected from the group consisting of a protonic acid capable of donating H⁺, a Lewis acid containing a halide ion and a mixture thereof.

6. The method according to claim 1, wherein the Pd in the main catalyst and the olefin are in a molar ratio of 1: (700-1000000); and
the dehydrating agent and the olefin are in a molar ratio of (0.5-8): 1.

7. The method according to claim 1, wherein the carbon monoxide and the olefin are in a molar ratio of (1-5): 1; and
the oxygen in the oxygen-containing atmosphere and the olefin are in a molar ratio of (0.5-3): 1.

8. The method according to claim 1, wherein the oxidative carbonylation reaction is performed at a temperature of 20-300°C under a pressure of 0.5-300 bar for a time of 0.2-40 hours.

9. The method according to claim 1, wherein the oxidative carbonylation reaction is performed in an alkyl alcohol solvent, with the alkyl alcohol solvent and the dehydrating agent being in a molar ratio of 10:1-0.1:10.

10. The method according to claim 1, wherein after the oxidative carbonylation reaction is completed, reaction product is separated by distillation under normal pressure or reduced pressure, and then residue from the distillation is used as recovered catalyst.
